# EUROPÄISCHE PATENTSCHRIFT

(11) **EP 1 948 311 B1**
(45) Veröffentlichungstag und Bekanntmachung des Hinweises auf die Patenterteilung: **16.06.2010**
(21) Anmeldenummer: 06818591.7
(22) Anmeldetag: 16.11.2006
(51) Int. Cl.: A61N 5/06, A61B 18/22

(54) **VORRICHTUNG ZUR MEDIZINISCHEN BEHANDLUNG VON PATIENTEN**
DEVICE FOR THE MEDICAL TREATMENT OF PATIENTS
DISPOSITIF DE TRAITEMENTS MEDICAL DE PATIENTS

(30) Priorität: 18.11.2005 DE 102005055523
(43) Veröffentlichungstag der Anmeldung: 30.07.2008
(73) Patentinhaber: Elexxion GmbH, 78315 Radolfzell (DE)
(72) Erfinder: SCHÄFER, Olaf, 78224 Singen (DE)
(74) Vertreter: Weiss, Peter
(86) Internationale Anmeldenummer: PCT/EP2006/011003
(87) Internationale Veröffentlichungsnummer: WO 2007/057185

(56) Entgegenhaltungen:
- WO-A-99/22667
- WO-A-03/103529
- DE-A1- 19 737 675
- DE-U1- 9 321 069
- US-A1- 2004 259 053

## Beschreibung

Die Erfindung betrifft eine Vorrichtung zur medizinischen Behandlung von Patienten mit einem Laserstrahl aus einem Halbleiter- bzw. Diodenlaser, indem der Laserstrahl in Form von Pulsen auf den zu behandelnden Bereich applizierbar ist und die Zeitdauer zwischen zwei aufeinander folgenden Pulsen länger als die Pulsdauer eines Einzelpulses ist.

### STAND DER TECHNIK

Lasersysteme stellen heute in der Medizin ein unverzichtbares Werkzeug dar. Sie ermöglichen ein präzises, punktgenaues und berührungsloses Arbeiten. Für medizinische Anwendungen gibt es eine Vielzahl von Lasersystemen. Von zentraler Bedeutung ist hierbei für jeden Laser sein aktives Medium, welches die Emissionswellenlänge vorgibt und somit das Einsatzgebiet des Lasers in der Medizin festlegt. Die Auswahl wird im wesentlichen durch die wollenlängenabhängige Absorption der Laserstrahlung im Gewebe getroffen.

Verschiedene Lasersysteme werden sowohl in der Humanmedizin, wie z.B. der Augenheilkunde, Dermatologie, plastischen Chirurgie, Gynäkologie, Neurochirurgie, Urologie und Zahnmedizin, als auch in der Tiermedizin eingesetzt. In der Zahnmedizin wird der Laser beispielsweise zur Behandlung von Parodontose und Zahnfleischerkrankungen sowie als Bohrer-Ersatz eingesetzt.

Grundlage der Erzeugung von Laserstrahlung ist immer die stimulierte Emission. Durch eine Anregung der Atome bzw. Moleküle im laseraktiven Medium erfolgt eine Besetzung höher gelegener Energieniveaus, welche für den Laserübergang verantwortlich sind. Ist die Anregung stark genug, um eine Übervölkerung des oberen Laserniveaus zu erzeugen (Pumpen), spricht man von einer Besetzungsinversion. Letztendlich kommt es, als Folge eines spontanen Emissionsüberganges zur stimulierten Emission, d.h. zu einer künstlich generierten Entvölkerung des oberen Laserniveaus und zur Abstrahlung von Laserstrahlen.

Das Verfahren, mit dem das Lasermedium angeregt wird, hängt vom verwendeten Lasermedium ab. Die drei wesentlichsten Anregungsarten sind
- Gasentladung, d.h., Plasmabildung bei Gaslasern
- optisches Pumpen bei Festkörperlasersystemen
- elektrisches Pumpen bei Diodenlaser

Bei den Diodenlasern werden Halbleiterkristalle als aktive Medien verwendet, die bei Anregung eine kohärente Strahlung im sichtbaren und im nahen infraroten Spektralbereich emittieren. In Halbleitern sind die Energiezustände der Elektronen nicht scharf, wie bei freien Atomen, sondern durch breite Bänder gegeben. Den Grundzustand bildet das Valenzband, den angeregten Zustand das Leistungsband. Die Anregung erfolgt üblicherweise am sogenannten pn-Übergang nach Anlegen einer äusseren Spannung. Die Elektronen werden vom Valenzband in das Leistungsband befördert, was zur Besetzungsinversion führt. Bei einer darauf folgenden stimulierten Emission kehren sie in das Valenzband zurück und senden dabei Licht aus. Die Emissionswellenlänge hängt vom energetischen Abstand zwischen Valenz- und Leistungsband ab, wobei sich der Bandabstand aus der Auswahl geeigneter Halbleiterverbindungen ergibt.

Diodenlaser werden in der Medizin seit Mitte der 90er Jahre eingesetzt. Die Hauptanwendungsgebiete sind Koagulation (Blutstillung), Chirurgie (Abtragen von Weichgewebe) und das Eliminieren von Krankheitserregern. Darüber hinaus können leistungsstarke Diodenlaser, wie sie beispielsweise in der DE 10 2004 006 932 beschrieben sind, auch zur Haarentfemung (Epilation) eingesetzt werden.

In der Zahnmedizin werden Diodenlaser für die chirurgische Schnittführung, die Abtötung von Keimen, die Softlasertherapie und zum Bleichen von Zähnen seit Mitte der 90er Jahre erfolgreich angesetzt. Während für alle benannten Anwendungen eine Leistung von 1 bis 3 Watt ausreichend ist, beeinflusst in der chirurgischen Schnittführung die Ausgangsleistung der Geräte die Schnittgeschwindigkeit. Hier ist somit eine höhere Ausgangsleistung wünschenswert. Die am Markt angebotenen Geräte erreichen jedoch nur eine Steigerung der Schnittgeschwindigkeit durch Ausgangsleistungen bis 15 Watt.

Diodenlaser sind in mehreren diskreten Wellenlängen erhältlich. Bekannt sind z.B. Wellenlängen von 635 nm (sichtbar, rot), 810 nm, 940 nm und 980 nm (alle Infrarot, unsichtbar) mit ausreichender Leistung für die beschriebenen Situationen. Darüber hinaus gibt es weitere Wellenlängen, die über eine ungenügende Ausgangsleistung für die beschriebenen Anwendungen besitzt.

In der Zahnmedizin werden hauptsächlich Diodenlaser von 810 nm und 980 nm eingesetzt.

Das Wirkungsprinzip dieser Diodenlaser am Patienten ist die Absorption des Laserlichts durch biologisches Gewebe. Die Absorption durch Wasser ist in diesem Wellenlängenbereich unerheblich (0,01% bis 0,1%). Der wesentliche Wirkmechanismus entsteht durch Absorption des Laserlichts durch Melanin (Haut) oder Hämoglobin (roter Blutfarbstoff). Die chirurgische Schnittführung mit einem Diodenlaser setzt also ein gut durchblutetes Gewebe voraus.

Anders als andere Lasersysteme (Gas- oder Festkörperlaser), welche in der Lage sind, optische Energie zu speichern und diese in einem kurzen Puls abzugeben, können Diodenlaser ähnlich wie eine Glühlampe, nur an- oder ausgeschaltet werden. Während ein Festkörperlaser wie ein Nd:YAG in kurzer Zeit (typischer Weise einige µs) einen Energieimpuls von mehr als ein Joule abgeben kann, der einer Spitzenleistung von mehreren 1000 Watt entspricht, kann der Diodenlaser nur mit einer maximalen Leistung (typischer Weise 2 bis 15 Watt) eingeschaltet werden. Geschieht dies über einen Zeitraum von einigen µs, wie beim Nd:YAG-Laser, werden auch nur Energien von einigen wenigen mJ erreicht. Diodenlaser werden daher in der medizinischen Anwendung meistens kontinuierlich (CW) oder mit relativ langen Quasi-Pulsen (einige ms) betrieben.

Für die thermische Wirkung auf das biologische Gewebe ist aber das zeitliche Energieabgabeverhalten enorm wichtig. Wird die Laserenergie in einem kurzen Puls abgegeben, ist die thermische Belastung für das Gewebe geringer. Bei extrem kurzen Pulsen im ns-fs-Bereich (Femtosekundenlaser) findet schliesslich überhaupt kein thermischer Energieübertrag auf das Gewebe mehr statt.

Beim Diodenlaser führt mit zunehmender Laserleistung, mit der sich zwar die Schnittgeschwindigkeit in der chirurgischen Anwendung steigern lässt, die thermische Belastung dazu, dass das Gewebe verbrennt. Dies wird als Carbonisierung bezeichnet und bedeutet: verkohltes Gewebe. Dadurch wird die Wundheilung gestört, es kommt zu toxischen Nebenprodukten. Der thermische Effekt der Carbonisierung limitiert also die maximal einsetzbare Laserleistung und damit die Schnittgeschwindigkeit.

Ein weiterer Aspekt der bisher bekannten Diodenlaser ist die schlechte Ankopplung der Laserstrahlung an das Gewebe. Je weniger Blutfarbstoff im Gewebe enthalten ist, desto weniger Laserstrahlung wird vom Gewebe absorbiert. In der zahnärztlichen Praxis ist dies häufig ein Problem, weil durch Anästhesiemittel die Durchblutung reduziert wird. Der Laserstrahl zeigt dann häufig keine oder nur eine schwache Reaktion auf dem bestrahlten Gewebe.

Aus der WO 99/22667 A ist allgemein eine Behandlung mit einem Halbleiterlaser bekannt. Dieser arbeitet bevorzugt im Bereich von 700 nm bis 1.100 nm. Dabei können die Lasersignale ständig oder gepulst ausgegeben werden.

Nach der US 2004/259053 A1 findet bei einer dentalen Behandlung ein Pulsdiodenlaser Anwendung, der in einem Bereich von 800 nm bis 980 nm mit einer Wiederholungsrate von 20 Hz bis 100 Hz, einer durchschnittlichen Leistung von 1 W bis 2 W und einer Pulsdauer von 1 msec. bis 5 msec. arbeitet. Wird bei einem derartigen Laser die Leistung erhöht, ist die Pulsdauer zu hoch, so dass es zu Verbrennungen kommt.

### AUFGABE

Aufgabe der vorliegenden Erfindung ist es, einen Diodenlaser für die medizinische Anwendung geeigneter zu machen

### LÖSUNG DER AUFGABE

Zur Lösung der Aufgabe führt, dass die Dauer eines einzelnen Pulses kleiner als 100.µs ist und der Laser zwischen den Pulsen mit einer Energie versorgt ist, welche unterhalb des Schwellwertes liegt, ab dem ein Laserbetrieb möglich ist.

D.h., mit einem Diodenlaser wird das Pulsverhalten von Festkörperlasern, wie dem Nd:YAG Laser simuliert, um die oben beschriebenen Effekte zu eliminieren.

Eine Voraussetzung für ein solches Diodenlasersystem ist eine Laserquelle, welche über eine ausreichende Leistung verfügt. Hierfür sollten wenigstens 5 Watt, vorzugsweise 25 bis 50 Watt Dauerleistung zur Verfügung stehen. Je höher die maximale Ausgangsleistung ist, desto höher ist die Pulsenergie in einem bestimmten Zeitintervall. Des weiteren muss die Laserquelle über die richtige Wellenlänge verfügen. Hier bietet sich der Bereich zwischen 700 bis 1.050 nm an. Vorzugsweise werden Laserquellen mit 810 nm eingesetzt, weil hier die Absorption in Hämoglobin sehr gut ist. Ähnliches gilt auch für eine Laserquelle mit einer Wellenlänge von 940 +/- 10 nm.

Die Einschaltzeit des Lasers sollte in einem Bereich von 2 bis 500 µs, vorzugsweise 10 bis 50 µs liegen und die Pausenzeit zwischen zwei Pulsen grösser als die Pulszeit, vorzugsweise 2 bis 5 Mal solang wie die Pulszeit sein, so dass eine chirurgische Schnittführung mit wesentlich erhöhter Geschwindigkeit ohne störende Carbonisierungseffekte erfolgt. Eine weitere Beobachtung ist, dass selbst eine Ankopplung an völlig anämisches, undurchblutetes Gewebe stattfindet.

Die elektronische Ansteuerung, welche den Strom für die Laserdiode bereitstellt, wird in einem bevorzugten Ausführungsbeispiel während des Betriebs permanent auf einen Wert eingestellt, welcher knapp unter dem Wert liegt, bei dem die Laserdiode anfängt Laserenergie zu erzeugen. Kurzzeitig wird die Steuerung veranlasst, den maximal zulässigen Strom der Laserdiode einzuschalten. Dieser Vorgang wird periodisch wiederholt.

Die besten Resultate können mit rechteckigen Pulsformen erreicht werden. Aufgrund von Beschränkungen der Stromversorgung sind rechteckige Pulsformen allerdings bei sehr kurzen Pulsen (< 50 µs) nicht möglich. Hier wird der Puls aufgrund der endlichen Anstiegs- und Abfallszeiten gaussförmig oder glockenförmig verlaufen. Ein wesentlicher Punkt für gaussförmige Pulse ist eine hohe Anstiegsflanke, um die maximale Pulsenergie schnell zu erreichen.

Um die Laserstrahlung zu applizieren, werden häufig Quarzfasern eingesetzt. Je kleiner der Durchmesser der Quarzfaser gewählt wird, desto grösser ist die Wirkung auf das Gewebe, weil die Leistungsdichte bei dünneren Fasern quadratisch zunimmt.

Zur Applikation des Laserlichtes befindet sich am distalen Ende der Übertragungsfaser ein Handstück, welches es dem Arzt ermöglicht, die Laserenergie gezielt zu applizieren. In einer besonderen Ausführungsform wird dieses Handstück zweiteilig ausgeführt und mit einer Kopplungsoptik versehen. Auf diese Weise sind Übertragungsfasern und Applikationsfasern getrennt.

Dadurch muss bei einer Beschädigung oder Verschmutzung nicht die gesamte Faser ausgetauscht werden, sondern nur der Applikationsteil.

Eine sinnvolle Ergänzung des beschriebenen Systems ist die Erweiterung um eine zweite Laserquelle, vorzugsweise eine Er:YAG Laserquelle. In der Zahnmedizin sind damit alle denkbaren Anwendungen durchführbar.

### FIGURENBESCHREIBUNG

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine diagrammartige Darstellung einer möglichen Ausführung der erfindungsgemässen Vorrichtung;
Figur 2 eine diagrammartige Darstellung einer weiteren möglichen Ausführungsform der erfindungsgemässen Vorrichtung;
Figur 3 eine weitere diagrammartige Darstellung einer möglichen Ausführungsform der erfindungsgemässen Vorrichtung.

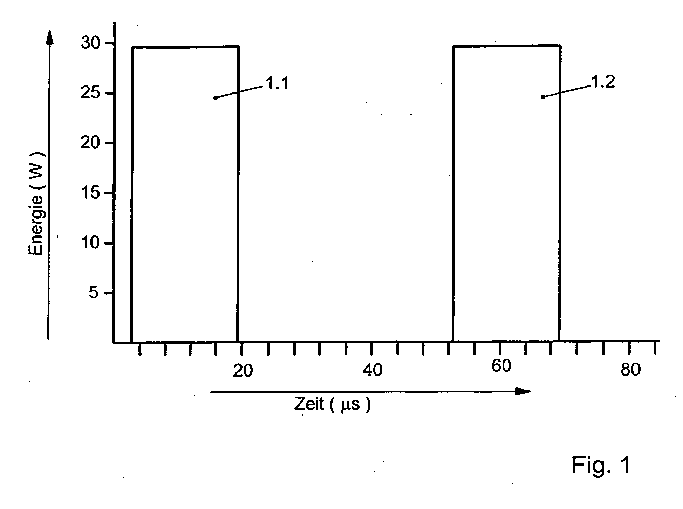

Gemäss Figur 1 ist die Energie, mit welcher ein erfindungsgemässer Diodenlaser betrieben wird in Watt, über der Zeit aufgezeigt. Dabei ist erkennbar, dass der Laserstrahl in Form von Pulsen 1.1 bzw. 1.2 ausgebracht wird. Zwischen den Pulsen 1.1 und 1.2 befindet sich ein pulsfreier Raum. Während dieser Zeit findet keine Applikation des Laserstrahls auf den zu behandelnden Bereich des Patienten statt. Die Pulse 1.1 und 1.2 werden mit etwa 30 Watt ausgebracht, die Dauer jedes Pulses 1.1 bzw. 1.2 beläuft sich auf ca. 16 µs und die Zeit zwischen den Pulsen 1.1 und 1.2 auf ca. 32 µs. Die Pulse haben eine weitgehend rechteckige Form.

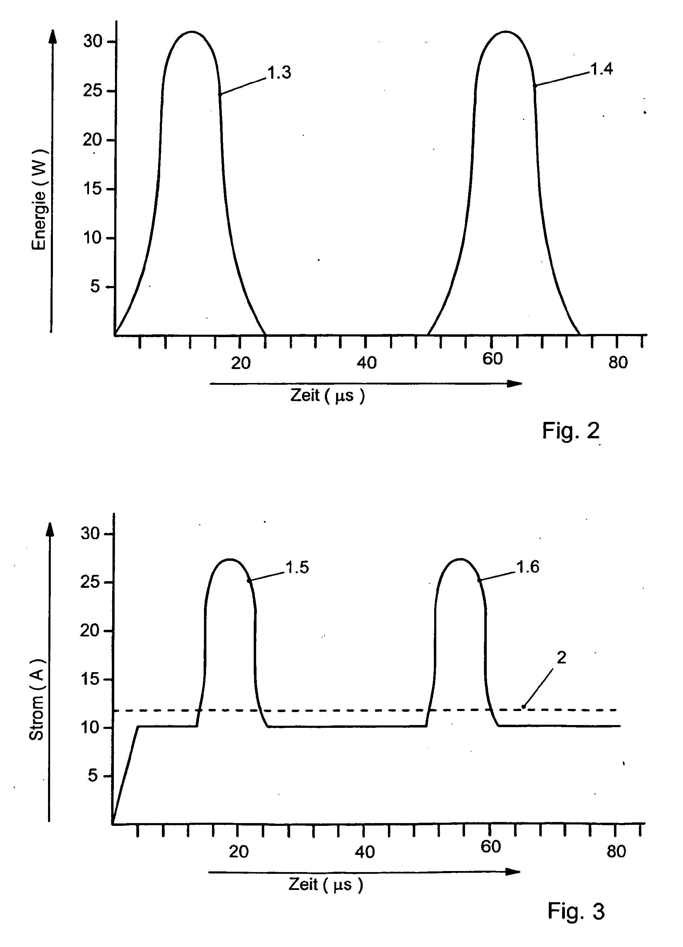

Da die rechteckige Form bei einem Diodenlaser nur schwer zu erreichen ist, dürfte in der Regel bei der erfindungsgemässen Vorrichtung die Pulse 1.3 und 1.4 in einer Form einer gausschen Kurve ausgegeben werden. Die ist in Figur 2 dargestellt. Der Anstieg soll auf jeden Fall mit mehr als 0,1 Watt pro µs erfolgen, so dass eine relativ steile Glockenkurve entsteht. Der Abfall verläuft ähnlich.

In Figur 3 ist eine besonders bevorzugte Ausführungsform der erfindungsgemässen Vorrichtung gezeigt. Hier wird der Strom bzw. die Energie immer kurz unterhalb eines Schwellwertes 2 gehalten, bei dem der Laser in Betrieb gehen würde. Dadurch wird die Zeit bis zum Ausbringen der Pulse 1.5 und 1.6 wesentlich verkürzt, so dass ein steilerer Anstieg der Glockenform der Kurve möglich ist, die sich schon beinahe der rechteckigen Form annähert.

## Patentansprüche

1. Vorrichtung zur medizinischen Behandlung von Patienten mit einem Laserstrahl aus einem Halbteiterlaser, indem der Laserstrahl in Form von Pulsen (1.1 bis 1.6) auf den zu behandelnden Bereich applizierbar ist und die Zeitdauer zwischen zwei aufeinander folgenden Pulsen länger als die Pulsdauer eines Einzelpulses ist,
**dadurch gekennzeichnet,**
**dass** die Dauer eines einzelnen Pulses (1.1 bis 1.6) kleiner als 100 µs ist und der Laser zwischen den Pulsen (1.5, 1.6) mit einer Energie versorgbar ist, welche unterhalb des Schwellwertes (2) liegt, ab dem ein Laserbetrieb möglich ist.

2. Vorrichtung nach Anspruch 1, **dadurch gekennzeichnet, dass** die Leistung eines einzelnen Pulses (1.1 bis 1.6) mindestens 10 W beträgt.

3. Vorrichtung nach Anspruch 1, **dadurch gekennzeichnet, dass** die Energieversorgung mindestens 50% des Schwellwertes (2) beträgt.

4. Vorrichtung nach wenigstens einem der Ansprüche 1 bis 3, **dadurch gekennzeichnet, dass** die Leistung beim Puls (1.1, 1.2) rechteckförmig ausgegeben wird.

5. Vorrichtung nach wenigstens einem der Ansprüche 1 bis 3, **dadurch gekennzeichnet, dass** die Leistung beim Puls (1.3, 1.4) gaussförmig mit einer Steigung von > 0,1 W/µs im Bereich der halben maximalen Pulsleistung ausgegeben wird.

6. Vorrichtung nach wenigstens einem der Ansprüche 1 bis 3, **dadurch gekennzeichnet, dass** die Leistung beim Puls dreieckig mit einer Steigung von > 0,1 W/µs ausgegeben wird.

7. Vorrichtung nach wenigstens einem der Ansprüche 1 bis 6, **dadurch gekennzeichnet, dass** dem Halbleiterlaser mit einer Wellenlänge zwischen 700 und 1.050 nm ein Übertragungsmedium zur Übertragung von Laserstrahlung auf eine Einrichtung zur Applikation der Laserstrahlung zugeordnet ist.

8. Vorrichtung nach Anspruch 7, **dadurch gekennzeichnet, dass** das Übertragungsmedium eine Quarzglasfaser ist.

9. Vorrichtung nach Anspruch 8, **dadurch gekennzeichnet, dass** die Faser einen Durchmesser von 100-400 µm aufweist.

10. Vorrichtung nach einem der Ansprüche 7 bis 9, **dadurch gekennzeichnet, dass** die Einrichtung zur Applikation der Laserstrahlung aus einem Rückteil und einem davon lösbaren Vorderteil besteht, wobei das Rückteil fest mit dem Übertragungsmedium verbunden ist und das Vorderteil auswechselbare Glasfasern aufnimmt.

11. Vorrichtung nach Anspruch 10, **dadurch gekennzeichnet, dass** das Rückteil wenigstens zwei optische Elemente, vorzugsweise Linsen, zur Einkopplung der Laserstrahlung in die Applikationsfaser aufweist.

12. Vorrichtung nach wenigstens einem der Ansprüche 7 bis 11, **dadurch gekennzeichnet, dass** dem Diodenlaser eine zweite Laserquelle zugeordnet ist.

13. Vorrichtung nach Anspruch 12, **dadurch gekennzeichnet, dass** die zweite Laserquelle ein Er:YAG-Laser ist.

14. Vorrichtung nach Anspruch 12 oder 13, **dadurch gekennzeichnet, dass** die zweite Laserquelle eine Wellenlänge von 2.940 nm aufweist.

15. Vorrichtung nach Anspruch 14, **dadurch gekennzeichnet, dass** der Er:YAG-Laser ein eigenes Übertragungsmedium und ein eigenes Applikationsteil besitzt.

16. Vorrichtung nach einem der Ansprüche 13 bis 15, **dadurch gekennzeichnet, dass** der Er:YAG-Laser ein eigenes Übertragungsmedium besitzt und ein gemeinsames Applikationsteil mit dem Diodenlaser.

## Claims

1. A device for the medical treatment of patients with a laser beam from a semiconductor laser, in that the laser beam can be applied to the region to be treated in the form of pulses (1.1 to 1.6) and the time between two successive pulses is longer than the pulse duration of an individual pulse,
**characterised in that**
the duration of an individual pulse (1.1 to 1.6) is less than 100 µs and the laser can be supplied between the pulses (1.5, 1.6) with an energy which lies beneath the threshold value (2) as from which laser operation is possible.

2. A device according to Claim 1, **characterised in that** the output of an individual pulse (1.1 to 1.6) is at least 10 W.

3. A device according to Claim 1, **characterised in that** the energy supply is at least 50% of the threshold value (2).

4. A device according to at least one of Claims 1 to 3, **characterised in that** the output upon the pulse (1.1, 1.2) is emitted in rectangular form.

5. A device according to at least one of Claims 1 to 3, **characterised in that** the output upon the pulse (1.3, 1.4) is emitted in a Gaussian shape with a gradient of > 0.1 W/µs in the region of the half-maximum pulse output.

6. A device according to at least one of Claims 1 to 3, **characterised in that** the output upon the pulse is emitted in triangular form with a gradient of > 0.1 W/µs.

7. A device according to at least one of Claims 1 to 6, **characterised in that** a transmission medium for transmitting laser radiation to an apparatus for applying the laser radiation is associated with the semiconductor laser with a wavelength of between 700 and 1050 nm.

8. A device according to Claim 7, **characterised in that** the transmission medium is a quartz-glass fibre.

9. A device according to Claim 8, **characterised in that** the fibre has a diameter of 100 - 400 µm.

10. A device according to one of Claims 7 to 9, **characterised in that** the apparatus for applying the laser radiation consists of a rear part and a front part detachable therefrom, the rear part being connected securely to the transmission medium and the front part receiving exchangeable glass fibres.

11. A device according to Claim 10, **characterised in that** the rear part has at least two optical elements, preferably lenses, for coupling the laser radiation into the application fibre.

12. A device according to at least one of Claims 7 to 11, **characterised in that** a second laser source is associated with the diode laser.

13. A device according to Claim 12, **characterised in that** the second laser source is an Er:YAG laser.

14. A device according to Claim 12 or 13, **characterised in that** the second laser source has a wavelength of 2940 nm.

15. A device according to Claim 14, **characterised in that** the Er:YAG laser has its own transmission medium and its own application part.

16. A device according to one of Claims 13 to 15, **characterised in that** the Er:YAG laser has its own transmission medium and a common application part with the diode laser.

## Revendications

1. Dispositif de traitement médical de patients, avec un faisceau laser d'un laser semi-conducteur, dans lequel le faisceau laser sous forme d'impulsions (1.1 à 1.6) peut être appliqué sur la zone à traiter et la durée entre deux impulsions successives est supérieure à la durée d'une impulsion,
**caractérisé par le fait que**
la durée d'une impulsion individuelle (1.1 à 1.6) est inférieure à 100 µs et le laser peut être alimenté, entre les impulsions (1.5, 1.6), par une énergie qui se situe au-dessous de la valeur de seuil (2) à partir de laquelle est possible un fonctionnement de laser.

2. Dispositif selon la revendication 1, **caractérisé par le fait que** la puissance d'une impulsion individuelle (1.1 à 1.6) est d'au moins 10 W.

3. Dispositif selon la revendication 1, **caractérisé par le fait que** l'alimentation en énergie est d'au moins 50% de la valeur de seuil (2).

4. Dispositif selon au moins l'une des revendications 1 à 3, **caractérisé par le fait que** la puissance à l'impulsion (1.1, 1.2) est sortie de forme rectangulaire.

5. Dispositif selon au moins l'une des revendications 1 à 3, **caractérisé par le fait que** la puissance à l'impulsion (1.3, 1.4) est sortie de forme gaussienne avec une montée de 0,1 W/µs à l'endroit de la demi-puissance d'impulsion maximale.

6. Dispositif selon au moins l'une des revendications 1 à 3, **caractérisé par le fait que** la puissance à l'impulsion est sortie de forme triangulaire avec une montée de 0,1 W/µs.

7. Dispositif selon au moins l'une des revendications 1 à 6, **caractérisé par le fait qu'**à chaque laser semi-conducteur avec une longueur d'onde comprise entre 700 et 1.050 nm est associé un support de transmission destiné à transmettre le rayonnement laser à un dispositif d'application du rayonnement laser.

8. Dispositif selon la revendication 7, **caractérisé par le fait que** le support de transmission est une fibre de verre de quartz.

9. Dispositif selon la revendication 8, **caractérisé par le fait que** la fibre présente un diamètre de 100 à 400 µm.

10. Dispositif selon l'une des revendications 7 à 9, **caractérisé par le fait que** le dispositif d'application du rayonnement laser se compose d'une partie arrière et d'une partie avant amovible de cette dernière, la partie arrière étant solidaire du support de transmission et la partie avant recevant des fibre de verre échangeables.

11. Dispositif selon la revendication 10, **caractérisé par le fait que** la partie arrière présente au moins deux éléments optiques, de préférence lentilles, pour le couplage du rayonnement laser dans la fibre d'application.

12. Dispositif selon au moins l'une des revendications 7 à 11, **caractérisé par le fait qu'**au laser à diodes est associée une deuxième source de laser.

13. Dispositif selon la revendication 12, **caractérisé par le fait que** la deuxième source de laser est un laser Er:YAG.

14. Dispositif selon la revendication 12 ou 13, **caractérisé par le fait que** la deuxième source de laser présente une longueur d'onde de 2.940 nm.

15. Dispositif selon la revendication 14, **caractérisé par le fait que** le laser Er:YAG possède un support de transmission propre et une partie d'application propre.

16. Dispositif selon l'une des revendications 13 à 15, **caractérisé par le fait que** le laser Er:YAG possède un support de transmission propre et une partie d'application commune avec le laser à diodes.
